# EUROPEAN PATENT APPLICATION

(11) **EP 3 741 361 A1**
(43) Date of publication of application: **25.11.2020**
(21) Application number: 20000178.2
(22) Date of filing: 09.05.2020
(51) Int. Cl.: A61K 9/50, A23D 9/007, A61K 31/05, A61K 9/00

(54) **GASTRO-RESISTANT FOOD/PHARMACEUTICAL CAPSULE FOR TREATMENT OF INFLAMMATORY BOWEL DISEASES**

(30) Priority: 09.05.2019 IT 201900006701
(71) Applicant: P&P Farma S.r.l., 10121 Torino (TO) (IT)
(72) Inventor: Bornoroni, Corrado, 00066 Maziana (RM) (IT); Di Renzo, Laura, 00183 Roma (RM) (IT); Gallese, Walter, 00166 Roma (RM) (IT); Giovinazzo, Corrado, I-10121 Torino (TO) (IT); Roversi, Francesco, I-10121 Torino (TO) (IT); Smeriglio, Antonella, I-10121 Torino (TO) (IT); Trombetta, Domenico, I-10121 Torino (TO) (IT); Seneci, Antonio, 20122 Milano (MI) (IT)
(74) Representative: Aprà, Mario

(57) **Abstract**

A gastro-resistant food/pharmaceutical capsule for treatment of inflammatory bowel diseases, provided with a gastro-resistant film coating, said coating Including:
- a gastro-resistant polymer film,
- a plasticizing means,
- a lubricating means, and
- an opacifying means,
wherein said capsule comprises:
- hydroxytyrosol, obtained from olive extract and/or olive leaves and/or chemical synthesis: between 2.5 and 20 mg/capsule;
- DL-alpha-tocopheryl acetate: between 3.75 and 30 mg/capsule;
- L-Menthol: between 25 and 300 mg/capsule,
said components being carried in olive and/or extra virgin olive oil.

## Description

### Technical field

The present invention relates to a gastro-resistant food/pharmaceutical capsule for treatment of inflammatory bowel diseases (IBD).

### Background art

Different types of gastro-resistant food/pharmaceutical capsules suitable, primarily or secondarily, for treatment of inflammatory bowel diseases (IBD), are already known in the state of the art.

A gastro-resistant food/pharmaceutical capsule of the type specified is known, by way of example, from the Italian patent application No. IT 102016000007757 [1], in the name of the same applicant.

In particular, IT 102016000007757 discloses a gastro-resistant food/pharmaceutical capsule comprising:

| | |
|---|---|
| hydroxytyrosol obtained from olive extract and/or olive leaves and/or chemical synthesis | between 2.5 and 20 mg/capsule *preferably* 7.5 mg/capsule |
| DL-alpha-tocopheryl acetate | between 3.75 and 30 mg/capsule *preferably* 10 mg/capsule |

The daily dose can consist of one or two capsules, according to clinical needs.

The capsule is in hard gelatine form including a gastro-resistant film coating, said coating comprising:
- a gastro-resistant polymer film,
- a plasticizing means,
- a lubricating means, and
- an opacifying means.

The coating prevents enzymatic hydrolysis process, so that hydroxytyrosol is not dispersed at gastric level and is thus only released at the level of the intestinal lumen, in this way guaranteeing maximum bioavailability.

Two clinical studies conducted by the inventors have shown an appreciable efficacy of the aforesaid formulation (in particular: hydroxytyrosol 7.5 mg/capsule + DL-alpha-tocopheryl acetate 10 mg/capsule) in order to reduce oxidative stress and cardiovascular risk [2, 3], as well as to improve liver damage correlated to Nonalcoholic fatty liver disease (NAFLD) in children (experimentation with the following doses: hydroxytyrosol 3.75 mg/capsule + DL-alpha-tocopheryl acetate 5 mg/capsule [4], without exhibiting any side effects.

It must also be noted how the administration of hydroxytyrosol carried in olive oil/extra virgin olive oil achieves a matrix effect that maximizes its absorption and enteric bioavailability. Hydroxytyrosol absorption at intestinal level is further enhanced by the presence of DL-alpha-tocopheryl acetate.

The presence of hydroxytyrosol, whose anti-inflammatory actions have been proven in the literature of the field [5, 6], also contributes to making the formulation according to IT 102016000007757 effective in the treatment of IBD.

In particular, it has been found that the aforesaid formulation achieves appreciable results in terms of promotion of the anti-inflammatory response and reduction of systemic inflammation, linked both to an evident decrease in the circulating levels of Interleukin-6 (IL-6), a major contributor of inflammatory processes, and to a significant increase in the levels of Interleukin-10 (IL-10), which is capable of stimulating proliferation of B lymphocytes and cell antibody production, inhibiting the cytokines responsible for the inflammatory process [7].

IBDs, such as primary ulcerative colitis and Crohn's disease, are characterised by conditions of chronic inflammation of the intestine with an estimated global prevalence of 10-15%, and are also considered risk factors for colorectal carcinoma. These diseases affect both sexes and have a clinical onset that is generally between 15 and 45 years of age [8].

Conventionally, IBDs are treated by pharmacological therapy with the administration of 5-aminosalicylic acid (mesalamine) and analogues, and glucocorticosteriods.

Similar treatments, even when effective, still exhibit considerable side effects.

On the other hand, treatment of IBDs with immunosuppressive therapies (anti TNF-α) is also known. However, these are treatments that, due to their evident side effects, are reserved solely for particularly severe cases.

Oxidative stress is considered one of the mechanisms underlying the pathophysiology of IBDs, since the inflammation is strictly connected to the formation of reaction intermediates, including reactive oxygen and nitrogen species (ROS/RNS). In this regard, experimental IBD studies on animal models show an increased production of reactive species already in the initial stages of the disease that correlate, among other things, with its severity and progression [9].

In the perspective of further improving the anti-inflammatory potentials of the capsule of IT 102016000007757 for the purpose of treating IBDs, at the same time overcoming the side effects of the aforesaid known pharmacological treatments, the inventors have taken into consideration the properties of mint essential oil, already widely evaluated as potential alternative therapy for the treatment of IBDs, due to its anti-inflammatory properties.

More in particular, it has been found that the main component of mint essential oil, i.e., L-Menthol, significantly contributes in obtaining the following benefits:
(i) antagonism at calcium channels level;
(ii) normalisation of the orocaecal transit time;
(iii) carminative effects;
(iv) agonism on the kappa opioid receptors;
(v) anti-infective and anti-inflammatory effects;
(vi) serotoninergic antagonism [10].

In this regard, a meta-analysis of 121 studies on chronic inflammatory bowel diseases showed how mint essential oil has an improved efficacy in the treatment of inflammatory diseases and chronic functional disorders of the intestinal tract compared to other remedies such as antispasmodics, tricyclic antidepressants and fibres [11]. The use of mint oil for the aforesaid purposes has been approved in Europe, and its use is now consolidated as first-line therapy [10].

Different types of formulations (e.g., in the form of gastro-resistant pearls or capsules) comprising mint oil are currently known in the state of the art to obtain an anti-inflammatory therapeutic action in the intestinal lumen.

However, said known formulations suffer from the side effects inevitably linked to the oral ingestion of an essential oil, such as heartburn, nausea and anal irritation [10].

On the other hand, from European patent application No. EP 2 070 545 A1 is know a formulation for the prevention and treatment of inflammatory disorders of the colon, with colon-specific release and to be taken orally, including:

| | |
|---|---|
| Mint oil | 10-1000 mg/day |
| *Curcuma Longa* or | 10-2000 mg/day |
| curcumin | 0.01-100 mg/day |
| *Olea europea*, or oleuropein and/or verbascoside | 10-2000 mg/day |
| and/or hydroxytyrosol | 0.1-500 mg/day |

Optionally, the capsule of EP 2 070 545 A1 can also include, in variable percentages, one or more of the following components:
- N-acetylcysteine: 0.1-1200 mg/day;
- glutathione: 0.1-250 mg/day;
- ubidecarenone: 0.1-500 mg;
- lactoferrin: 0.1-250 mg/day;
- carotenoids: 0.1-25 mg/day;
- polyphenols: 0.1-3000 mg/day;
- vitamin C: 0.1-500 mg/day;
- vitamin E: 0.1-500 mg/day;
- St. John's Wort extract or a derivative thereof: 0.1-7200 mg/day;
- kava kava extract or a derivative thereof: 0.1-500 mg/day;
- valerian extract or a derivative thereof: 0.1-1500 mg/day;
- saffron extract or a derivative thereof: 0.1-2500 mg/day;
- camomile extract or a derivative thereof: 0.1-2500 mg/day;
- passionflower extract or a derivative thereof: 0.1-2500 mg/day;
- griffonia extract or a derivative thereof: 0.1-2500 mg/day.

The synergy between the extracted derivatives of Curcuma Longa and Olea Europea with mint essential oil cooperates in achieving a local colon-specific anti-inflammatory action.

On the other hand, the colon-specific release property of the capsule of EP 2 070 545 A1 makes it possible to significantly overcome the problem of gastroesophageal reflux or gastritis related to the oral ingestion of mint essential oil.

However, it can be appreciated how said known colon-specific capsule is able to obtain only a strictly local colon-specific anti-inflammatory action, and is hence ineffective for the treatment of inflammatory diseases that involve different tracts of the intestinal lumen.

Moreover, it has been found that said anti-inflammatory action, obtained through synergy between extractive derivatives of Curcuma Longa and Olea Europea with mint essential oil, appears open to improvement.

### Technical problem

In the light of the above, the object of the present invention is to provide a gastro-resistant food/pharmaceutical capsule to be used in treatment of inflammatory bowel diseases (IBD), which has an improved efficacy in promoting the anti-inflammatory response and in reducing systemic inflammation.

On the other hand, it is also an object of the present invention to provide a gastro-resistant food/pharmaceutical capsule as specified, which guarantees optimal absorption and bioavailability of L-Menthol in the intestinal tract without gastric dispersion, and which makes it possible to obtain appreciable anti-inflammatory actions with low doses of L-Menthol.

### Solution to the problem

In view of the above, the present invention provides a gastro-resistant food/pharmaceutical capsule for treatment of inflammatory bowel diseases (IBD) according to claim 1.

Further advantageous features of the invention are included in the dependent claims.

### Brief description of the drawings

The features and advantages of the present invention will be more apparent from the detailed description of an embodiment, provided below with reference to the accompanying drawings, wherein:

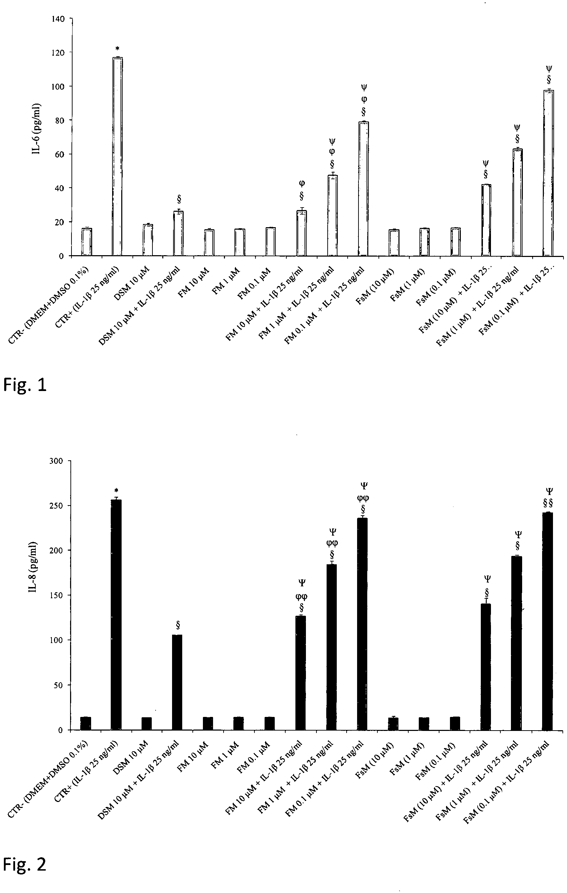

- Fig. 1 is a graphic representation illustrating the release of IL-6 (pg/ml) following inflammation of the Caco-2 cells induced with IL-1β 25 ng/ml. **P*<0.001 *vs* CTR-; ^{§}*P*<0.001 *vs* CTR+; ^{ϕ}*P*<0.001 *vs* FsM + IL-1β 25 ng/ml; ^{ψ}*P*<0.001 *vs* DSM + IL-1β 25 ng/ml in a comparative experimental test regarding a formulation comprising hydroxytyrosol (7.5 mg) and DL-alpha-tocopheryl acetate (10 mg) with, respectively without, the addition of L-Menthol, compared with an anti-inflammatory reference medicine (dexamethasone (DSM 10 µM));
- Fig. 2 is a graphic representation illustrating the release of IL-8 (pg/ml) following inflammation of the Caco-2 cells induced with IL-1β 25 ng/ml. **P*<0.001 *vs* CTR-; ^{§}*P*<0.001 *vs* CTR+; *^{§§}P*<0.05 *vs CTR*+; ^{ϕϕ}*P*<0.05 *vs* FsM + IL-1β 25 ng/ml; ^{ψ}*P*<0.001 *vs* DSM + IL-1β 25 ng/ml, in the same experimental test of Fig. 1;

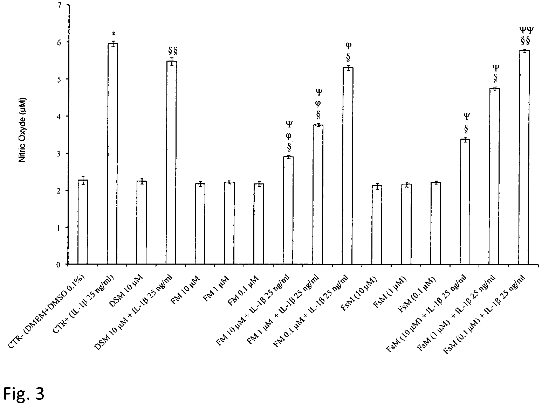

- Fig. 3 is a graphic representation illustrating the release of NO (µM) following inflammation of the Caco-2 cells induced with IL-1β 25 ng/ml. **P*<0.001 *vs* CTR-; ^{§}*P*<0.001 *vs* CTR+; ^{§§}*P*<0.05 *vs* CTR+; ^{ϕ}*P*<0.001 *vs* FsM + IL-1β 25 ng/ml; ^{ψ}*P*<0.001 *vs* DSM + IL-1β 25 ng/ml; ^{ψψ}P<0.05 *vs* DSM + IL-1β 25 ng/ml, in the same experimental test of Fig. 1.

### Detailed description of the invention

The description of an embodiment of the gastro-resistant food/pharmaceutical capsule for treatment of inflammatory bowel diseases (IBD) according to the present invention is provided in the following.

In particular, the invention relates to a gastro-resistant food/pharmaceutical capsule for treatment of inflammatory bowel diseases, which includes:
- a gastro-resistant polymer film,
- a plasticizing means,
- a lubricating means, and
- an opacifying means.

In particular, according to the invention, the capsule comprises the following components, carried in olive and/or extra virgin olive oil:

| | |
|---|---|
| hydroxytyrosol, obtained from olive extract and/or olive leaves and/or chemical synthesis | between 2.5 and 20 mg/capsule |
| DL-alpha-tocopheryl acetate | between 3.75 and 30 mg/capsule |
| L-Menthol | between 25 and 300 mg/capsule |

Advantageously, in an embodiment of the present invention, the capsule comprises the following components, carried in olive and/or extra virgin olive oil:

| | |
|---|---|
| hydroxytyrosol, obtained from olive extract and/or olive leaves and/or chemical synthesis | 7.5 mg/capsule |
| DL-alpha-tocopheryl acetate | 10 mg/capsule |
| L-Menthol | between 25 and 300 mg/capsule |

According to a further embodiment of the present invention, the capsule comprises the following components, carried in olive and/or extra virgin olive oil:

| | |
|---|---|
| hydroxytyrosol, obtained from olive extract and/or olive leaves and/or chemical synthesis | between 2.5 and 20 mg/capsule |
| DL-alpha-tocopheryl acetate | between 5 and 10 mg/capsule |
| L-Menthol | between 25 and 100 mg/capsule |

On the other hand, according to a preferred embodiment of the invention, the capsule comprises the following components, carried in olive and/or extra virgin olive oil:

| | |
|---|---|
| hydroxytyrosol, obtained from olive extract and/or olive leaves and/or chemical synthesis | 7.5 mg/capsule |
| DL-alpha-tocopheryl acetate | 10 mg/capsule |
| L-Menthol | between 25 and 100 mg/capsule |

The daily dose for an adult can consist of one or two capsules, according to clinical needs.

For paediatric use, administering of a reduced dose is provided and, in particular, a capsule comprises the following components, carried in olive and/or extra virgin olive oil:

| | |
|---|---|
| hydroxytyrosol, obtained from | 3.75 mg/capsule |
| olive extract and/or olive leaves and/or chemical synthesis | |
| DL-alpha-tocopheryl acetate | 5 mg/capsule |
| L-Menthol | between 25 and 100 mg/capsule |

As is further apparent on the basis of the results of the experimental test set forth below, the interaction of hydroxytyrosol and DL-alpha-tocopheryl acetate with L-Menthol allows obtaining a surprising synergic effect in terms of increase of efficacy in the treatment of inflammatory bowel diseases.

In addition, the formulation according to the present invention allows achieving the aforesaid synergic effects while maintaining the amount of L-Menthol within an essentially limited range, thus preventing the risk of an opposite proinflammatory effect linked to the excessive presence of L-Menthol in the formulation.

It is also found that the claimed formulation allows significantly improving the release of L-Menthol in the intestinal lumen, guaranteeing its better absorption.

On the other hand, by carrying the L-Menthol in olive and/or extra virgin olive oil it is possible to optimise the bioavailability of L-Menthol in the intestinal lumen, at the same time lessening the impact of L-Menthol on the walls of the intestinal lumen.

### Experimental test

### Overview

An experimental test of comparative type was conducted on a transwell *in vitro* model of Caco-2 cells, taking into consideration:
(a) a formulation (hereinafter FsM) comprising:
   hydroxytyrosol, obtained from standardised olive extract, or from synthesis: 7.5 mg, and
   DL-alpha-tocopheryl acetate: 10 mg;
(b) a formulation (hereinafter FcM) comprising:
   hydroxytyrosol, obtained from standardised olive extract, or from synthesis: 7.5 mg, and
   DL-alpha-tocopheryl acetate: 10 mg;
with the addition of L-Menthol in a range from 25 - 100 mg, subsequently compared with an anti-inflammatory reference medicine (dexamethasone (DSM 10 µM));

The Caco-2 cells, isolated from human colorectal adenocarcinoma, were grown on specific porous media for a total of 21 days in order to cause them to polarise and differentiate forming a continuous epithelium (monolayer), with functional tight junctions and apical microvilli (brush border). The cell monolayer contributes in defining a physical and biochemical barrier to the passage of ions and small molecules, and separates the model into two distinct compartments, namely:
- an apical compartment, which mimics the intestinal lumen, *in vivo*, and
- a basolateral compartment, which mimics the blood and lymphatic circulation compartment of the organism.

These cells express receptors and enzymes normally associated with the brush border of the enterocytes as efflux carriers and proteins, acting, both from a morphological and functional viewpoints, as mature enterocytes.

### Conduction of the test

After having tested the formulations FcM and FsM (i.e., with and without L-Menthol, respectively) in the predetermined range of concentrations (0.1-10 µM), as well as the anti-inflammatory reference medicine dexamethasone (DSM 10 µM), and after having conducted the negative control (DMEM + DMSO 0.1%), to verify a possible proinflammatory effect of the same, inflammation was induced by treating the cell monolayer with proinflammatory agent interleukin (IL) IL-1β 25 ng/ml for 24 h (positive control) [12].

Specifically, in order to assess the anti-inflammatory activity of the formulations FcM and FsM in relation to the anti-inflammatory reference medicine, the following co-treatments were carried out:
- DSM 10 µM + IL-1β 25 ng/ml;
- FM (0.1-1.10 µM) + IL-1β 25 ng/ml;
- FsM (0.1-1.10 µM) + IL-1β 25 ng/ml;

At the end of the incubation period (24 hours), the inflammatory markers IL-6, IL-8 and nitric oxide (NO) released in the cell culture medium were dosed, by means of immunoenzymatic assay (ELISA).

### Results

The results of the aforesaid test are illustrated graphically in the enclosed Figs. 1-3.

As can be seen based on the aforesaid Figs. 1-3, the dexamethasone as well as the two formulations with and without L-Menthol (FcM and FsM respectively), did not cause the release of dosed inflammatory markers (IL-6, IL-8 and NO). In fact, no statistically significant difference was observed compared to the negative control (CTR-).

Therefore, it is possible to conclude that the treatments have no proinflammatory effect on the intestinal barrier.

On the contrary, it was observed how the treatment with IL-1β at the concentration of 24 ng/ml (CTR+) caused a statistically significant release (P<0.001) of all the inflammatory markers taken into consideration (IL-6, IL-8 and NO), where compared to the negative control (CTR-).

As apparent from Figs. 1-3, the co-treatment with the formulation without the addition of L-Menthol (FsM) already makes it possible obtaining a statistically significant and dose-dependent reduction of the release of IL-6 (*P*<0.001), IL-8 (*P*<0.001 and *P*<0.05 at the concentration of 0.1 µM) and NO (*P*<0.001 and *P*<0.05 at the concentration of 0.1 µM) compared to the CTR+.

On the other hand, by analyzing the results relating to the formulation with the addition of L-Menthol (FcM), it has been possible appreciating not only a statistically significant and dose-dependent reduction of all the inflammatory markers analysed (*P*<0.001) compared to the CTR+, but also a statistically significant reduction compared to the formulation without L-Menthol (FsM) with a *P*<0.001 with regard to the release of IL-6 and NO and with a *P*<0.05 with regard to the release of IL-8.

It is also observed that the formulation containing L-Menthol at the concentration of 10 µM proved just as effective in countering the release of IL-6 as dexamethasone tested at the same concentration (10 µM) (Fig. 1).

Moreover, both the formulations, with and without the addition of L-Menthol (FcM, FsM, respectively) proved more effective than dexamethasone in countering the release of nitric oxide (Fig. 3) with a *P*<0.001 at the concentrations of 1 and 10 µM.

Cell viability (MTT assay) as well as membrane resistivity (TEER) and paracellular flux (via fluorescent probe) were determined at the end of all the treatments, showing that they did not alter the cell viability and the barrier properties of the cell monolayer.

The effects observed are thus specifically and unequivocally attributable to the observed anti-inflammatory activity.

### Conclusions:

- the addition of L-Menthol in a formulation comprising hydroxytyrosol obtained from standardised olive extract or from synthesis (7.5 mg) and DL-alpha-tocopheryl acetate (10 mg) allows obtaining a surprising synergic effect in terms of increase of efficacy in the treatment of inflammation caused by IL-1β;
- the aforesaid effects can also be observed at low concentrations of L-Menthol (0.1-10 µM);
- in the range of tested doses (0.1-10 µM), the addition of L-Menthol to the specified formulation does not give rise to any side effects of proinflammatory type, nor to alterations of cell viability.

As can be seen from the above, the present invention allows achieving the objects set forth in the introduction in a simple and advantageous manner.

### Bibliography

1. Italian patent application No. 102016000007757, with the title "Capsula alimentare/farmaceutica gastroresistente per il contenimento e la somminstrazione di idrossitirosolo", filed on 26 January 2016 by the same applicant.
2. Colica C. et al., Oxid Med Celi Longev. 2017; 2017:2473495
3. Di Renzo L. et al., Biomedicine & Prevention. 2017 Vol 1. Doi: 10.19252/00000005C
4. Nobili V. et al., Antioxid Redox Signal. 2018 Dec 27. doi: 10.1089/ars. 2018.7704
5. "Hydroxytyrosol: Bioavailability, toxicity, and clinical applications" (accessible from: https:/www.ncbi.nlm.nih.gov/pubmed/29433260)
6. "Hydroxytyrosol: A natural compound with promising pharmacological activities" (accessible from: https:/www.ncbi.nlm.nih.gov/pubmed/31884046)
7. "The anti-inflammatory effects of Hydroxytyrosol and Vitamin E on paediatric NAFLD" (accessible from: https:/www.dldjournalonline.com/article/S1590-8658(19)31088-6/fulltext)
8. Kaplan G.G. Nat Rev Gastroenterol Hepatol. 2015; 12(12):720-272
9. Tian T., Wang Z,. Zhang J. Oxid Med Celi Longev. 2017;2017:4535194
10. Wedlake L. et al., Inflamm Bowel Dis 2014; 20: 576-586
11. Enck P. et al., EurJ Gastroenterol Hepatol. 2010; 22:1402-1411
12. Tesoriere et al., Br J Nutr. 2014 Feb;111(3):415-23.

## Claims

1. A gastro-resistant food/pharmaceutical capsule for treatment of inflammatory bowel diseases, provided with a gastro-resistant film coating, said coating including:
- a gastro-resistant polymer film,
- a plasticizing means,
- a lubricating means, and
- an opacifying means,
wherein said capsule comprises:
- hydroxytyrosol, obtained from olive extract and/or olive leaves and/or chemical synthesis: between 2.5 and 20 mg/capsule;
- DL-alpha-tocopheryl acetate: between 3.75 and 30 mg/capsule;
- L-Menthol: between 25 and 300 mg/capsule,
said components being carried in olive and/or extra virgin olive oil.

2. The food/pharmaceutical capsule according to claim 1, comprising:
- hydroxytyrosol, obtained from olive extract and/or olive leaves and/or chemical synthesis: 7.5 mg/capsule;
- DL-alpha-tocopheryl acetate: 10 mg/capsule;
- L-Menthol: between 25 and 300 mg/capsule,
said components being carried in olive and/or extra virgin olive oil.

3. The food/pharmaceutical capsule according to claim 1, comprising:
- hydroxytyrosol, obtained from olive extract and/or olive leaves and/or chemical synthesis: between 2.5 and 20 mg/capsule;
- DL-alpha-tocopheryl acetate: between 5 and 10 mg/capsule;
- L-Menthol: between 25 and 100 mg/capsule,
said components being carried in olive and/or extra virgin olive oil.

4. The food/pharmaceutical capsule according to claim 1, comprising:
- hydroxytyrosol, obtained from olive extract and/or olive leaves and/or chemical synthesis: 7.5 mg/capsule;
- DL-alpha-tocopheryl acetate: 10 mg/capsule;
- L-Menthol: between 25 and 100 mg/capsule,
said components being carried in olive and/or extra virgin olive oil.

5. The food/pharmaceutical capsule according to claim 1, comprising:
- hydroxytyrosol, obtained from olive extract and/or olive leaves and/or chemical synthesis: 3.75 mg/capsule;
- DL-alpha-tocopheryl acetate: 5 mg/capsule;
- L-Menthol: between 25 and 300 mg/capsule,
said components being carried in olive and/or extra virgin olive oil.
